# FASCICULE DE BREVET EUROPEEN

(11) **EP 1 608 978 B1**
(45) Date de publication et mention de la délivrance du brevet: **20.08.2008**
(21) Numéro de dépôt: 04742379.3
(22) Date de dépôt: 26.03.2004
(51) Int. Cl.: G01N 33/558, G01N 33/543

(54) **PROCEDES IMMUNOCHROMATOGRAPHIQUES EN PHASE SOLIDE**
FESTPHASE IMMUNOCHROMATOGRAPHISCHE TESTVERFAHREN
SOLID-PHASE IMMUNOCHROMATOGRAPHIC METHODS

(30) Priorité: 28.03.2003 FR 0303877
(43) Date de publication de la demande: 28.12.2005
(73) Titulaire: Vedalab, 61000 Alençon (FR)
(72) Inventeur: DONATI, Raphaél, F-61250 Radon (FR); BIGOT, Patrick, F-61150 Ecouche (FR)
(74) Mandataire: Hinterberg, Katherine
(86) Numéro de dépôt international: PCT/FR2004/000775
(87) Numéro de publication internationale: WO 2004/088320

(56) Documents cités:
- EP-A- 0 458 231
- EP-A- 0 462 376
- EP-A- 1 020 726
- WO-A-97/09620
- WO-A-99/36780
- US-A- 6 008 056

## Description

La présente invention concerne un procédé de détection d'un analyte dans un échantillon liquide.

Les tests immunochromatographiques en phase solide sont bien connus de l'homme du métier. Ces tests mettent en oeuvre un support solide poreux au sein duquel l'échantillon et les réactifs migrent par diffusion capillaire. On connaît notamment les dispositifs dans lesquels le support solide se présente sous la forme d'un «dip-stick». Ces tests utilisent un support solide chromatographique comportant une zone de détection sur laquelle est immobilisé un réactif de capture spécifique de l'analyte. Ce support solide est mis en contact avec une solution comprenant d'une part l'échantillon à tester et d'autre part un réactif de liaison marqué spécifique de l'analyte. Cette solution migre par diffusion capillaire dans le support solide jusqu'à la zone portant le réactif de capture immobilisé. Dans un test sandwich, le réactif de liaison marqué se lie à l'analyte alors que ce dernier est immobilisé sur le support solide par le réactif de capture. La présence ou l'absence de l'analyte dans l'échantillon est ainsi mesurée par la détection du réactif marqué.

EP 0 284 232 décrit des tests immunochromatographiques en phase solide dans lesquels le support solide porte directement, sous forme lyophilisée ou déshydratée, le réactif de liaison conjugué à un marqueur particulaire. Le réactif conjugué avec le marqueur particulaire est immobile sous forme lyophilisée mais devient mobile dans le support solide à l'état humide. Ainsi, lorsque le support solide est mis en contact avec un échantillon liquide, ce dernier migre par diffusion capillaire dans le support entraînant le réactif de liaison conjugué au marqueur particulaire. Dans ces tests, il n'est pas nécessaire de mélanger préalablement le réactif conjugué et l'échantillon et tous les réactifs nécessaires au test sont donc intégrés au support solide. De plus, le réactif de liaison marqué spécifique de l'analyte est marqué avec un marqueur particulaire détectable par observation directe: Aucune manipulation supplémentaire n'est donc nécessaire pour lire le résultat du test. Ces tests ne nécessitent donc que très peu de manipulations et sont d'un usage facile et rapide.

EP 0 291 194, EP 0 560 411 et EP 0 560 410 décrivent également des dispositifs de test dans lesquels le réactif de liaison conjugué à un marqueur particulaire est porté par le support solide. De plus dans ces dispositifs, le support solide est incorporé dans un boîtier pourvu d'une ouverture pour le dépôt de l'échantillon et d'une fenêtre d'observation pour la lecture des résultats. Le boîtier facilite la préhension du dispositif et protége le support solide. En outre, ces brevets décrivent également des dispositifs dans lesquels l'une des extrémités du support solide est saillante par rapport au boîtier pour faciliter le dépôt de l'échantillon liquide. Cette extrémité saillante du support solide peut ainsi être directement mise en contact avec un flux d'urine par exemple.

WO 00/00288 décrit des dispositifs améliorés comprenant un boîtier et un support solide. Le support solide étant pourvu d'un organe de captation mobile pour une meilleure collecte de l'échantillon.

EP-A1-0 458 231 concerne des tests immunologiques en phase solide pour la détection d'un analyte dans un échantillon liquide. Ces tests mettent en oeuvre un support solide, typiquement une membrane, sur laquelle est immobilisé un réactif de capture de l'analyte. Après dépôt de l'échantillon, l'analyte immobilisé sur le support solide est détecté à l'aide d'un réactif de liaison conjugué à l'uréase. Ce document décrit des procédés dans lesquels l'échantillon puis le réactif de liaison marqué sont déposés sur le support solide.

EP-A2-0 462 376 décrit des tests immunochromatographiques à migration latérale. Les test décrits mettent en oeuvre un réactif de liaison conjugué à un marqueur particulaire. Ce document décrit des tests dans lesquels l'échantillon et le réactif de liaison conjugué sont appliqués séparément sur le support solide ou combinés préalablement au dépôt sur le support pour former une solution test. De préférence, le réactif de liaison conjugué est incorporé au support chromatographique.

US 6,008,056 décrit des dispositifs automatisés pour l'immunochromatographie latérale. Le réactif de liaison marqué est incorporé au support solide ou mélangé avec l'échantillon avant ou lors du dépôt sur le support chromatographique.

EP-A1-1 020 726 décrit des tests immunochromatographiques à migration latérale. L'échantillon est déposé sur le support avant le réactif de liaison marqué ou un mélange comprenant l'échantillon et un ou plusieurs réactifs marqués est déposé sur le support.

WO-A1-97 09620 décrit des procédés de détection d'un analyte dans un échantillon par immunochromatographie quantitative ou semi quantitative. Le réactif de liaison marqué est incorporé au support solide.

Cependant, ces tests immunochromatographiques en phase solide présentent parfois une sensibilité et une reproductibilité insuffisantes. Ce problème se pose plus particulièrement pour la détection d'analytes présents à une faible concentration ou pour la détection d'analytes dans un échantillon de nature complexe tel que du sang total par exemple. De plus, en raison de ces déficiences de tels tests ne conviennent que pour déterminer l'absence ou la présence d'un analyte dans un échantillon et ne permettent donc que l'obtention d'un résultat qualitatif. Des mesures plus quantitatives ne sont que difficilement réalisables. En outre, on observe fréquemment un bruit de fond important ainsi qu'un effet de zone (ou « Hook effect ») qui altèrent la lisibilité du résultat. L'effet de zone est un effet indésirable bien connu dans les tests immunologiques. Il se produit lorsque l'analyte est présent dans l'échantillon à une concentration très élevée. L'effet de zone peut alors conduire à un résultat négatif concluant de façon aberrante à l'absence de l'analyte dans l'échantillon.

Pour remédier à ces inconvénients la présente invention propose des procédés immunochromatographiques en phase solide permettant d'obtenir une sensibilité et une reproductibilité accrues tout en limitant le bruit de fond et les effets de zone. Les procédés selon l'invention sont particulièrement adaptés à des échantillons de nature complexe tels que du sang par exemple. Etant donné que le bruit de fond est diminué alors que la sensibilité et la reproductibilité sont accrues, les procédés de la présente invention, permettent avantageusement de détecter plusieurs analytes différents de façon simultanée sur le même support. De plus, l'analyte présent dans l'échantillon liquide peut être mesuré et quantifié grâce aux performances des procédés selon l'invention.

Dans les procédés selon la présente invention, le réactif de liaison conjugué à un marqueur particulaire est ajouté extemporanément sous forme liquide. Ainsi, dans les procédés selon l'invention l'ordre de dépôt de l'échantillon et des divers réactifs revêt une grande importance pour la performance du test de détection.

Dans un premier mode de réalisation, le procédé de détection d'un analyte dans un échantillon liquide selon l'invention comprend les étapes suivantes :
a) on dispose d'un support solide poreux pourvu d'une zone de collection et d'une zone de détection, un réactif de capture spécifique dudit analyte étant immobilisé dans la zone de détection ;
b) on dépose, séparément, successivement et extemporanément, dans la zone de collection du support solide poreux:
   i) un réactif de liaison conjugué susceptible de se lier spécifiquement avec l'analyte ou avec le réactif de capture à un marqueur particulaire, le réactif étant sous forme liquide,
   ii) l'échantillon liquide,
c) on attend un temps suffisant pour la migration par diffusion capillaire du réactif de liaison conjugué à un marqueur particulaire et de l'échantillon liquide, depuis la zone de collection jusqu' à la zone de détection du support solide poreux,
d) on observe la mesure dans laquelle le réactif conjugué à un marqueur particulaire se fixe dans la zone de détection.

Avantageusement, à l'étape b) on dépose l'échantillon liquide en amont du réactif de liaison conjugué à un marqueur particulaire, par rapport au sens de migration depuis la zone de collection jusqu' à la zone de détection du support solide poreux.

La présente invention concerne également un procédé de détection d'un analyte dans un échantillon liquide comprenant les étapes suivantes :
a) on dispose d'un support solide poreux pourvu d'une zone de collection et d'une zone de détection, un réactif de capture étant immobilisé dans la zone de détection ;
b) on dépose, séparément, successivement et extemporanément, dans la zone de collection du support solide poreux :
   i) un réactif de liaison conjugué à un marqueur particulaire, le réactif étant sous forme liquide,
   ii) l'échantillon liquide,
   iii) un diluant sous forme liquide,
c) on attend un temps suffisant pour la migration par diffusion capillaire du réactif conjugué à un marqueur particulaire, de l'échantillon liquide et du diluant, depuis la zone de collection jusqu'à la zone de détection du support solide poreux,
d) on observe la mesure dans laquelle le réactif de liaison conjugué à un marqueur particulaire se fixe dans la zone de détection.

Avantageusement, à l'étape b) on dépose le diluant sous forme liquide en amont du réactif de liaison conjugué à un marqueur particulaire et en amont de l'échantillon liquide, par rapport au sens de migration depuis la zone de collection jusqu' à la zone de détection du support solide poreux.

Dans un mode de réalisation préféré de l'invention, le réactif de liaison conjugué à un marqueur particulaire et le réactif de capture immobilisé dans la zone de détection permettent de détecter l'analyte par un test sandwich.

Dans un autre mode de réalisation préféré de l'invention, le réactif de liaison conjugué à un marqueur particulaire et le réactif de capture immobilisé dans la zone de détection permettent de détecter l'analyte par un test de compétition.

Préférentiellement, la zone de détection du support solide poreux comporte un deuxième réactif de capture immobilisé en aval du premier réactif de capture.

Préférentiellement, le support solide poreux est un support solide poreux en forme de bande ou de bandelette chromatographique.

Avantageusement, le support solide poreux est intégré dans un support de préhension pourvu d'au moins une fenêtre d'observation permettant d'observer la mesure dans laquelle le réactif conjugué à un marqueur particulaire se fixe dans la zone de détection du support solide poreux.

Dans un mode de réalisation particulier de l'invention, le support de préhension est pourvu d'au moins une ouverture permettant respectivement le dépôt de l'échantillon liquide, du réactif de liaison conjugué à un marqueur et, le cas échéant, du diluant, dans la zone de collection du support solide poreux.

Dans un mode de réalisation avantageux de l'invention, le support solide poreux est intégré dans un support de préhension pourvu d'au moins une fenêtre d'observation permettant d'observer la mesure dans laquelle le réactif conjugué à un marqueur particulaire se fixe dans la zone de détection du support solide poreux ; le support solide poreux étant également pourvu d'une première ouverture permettant le dépôt dans la zone de collection du support solide poreux, du réactif de liaison conjugué à un marqueur particulaire, et d'une deuxième ouverture, en amont de la première ouverture, permettant le dépôt dans la zone de collection du support solide poreux, de l'échantillon liquide.

Dans un autre mode de réalisation avantageux de l'invention, le support solide poreux est intégré dans un support de préhension pourvu d'au moins une fenêtre d'observation permettant d'observer la mesure dans laquelle le réactif conjugué à un marqueur particulaire se fixe dans la zone de détection du support solide poreux ; le support de préhension étant également pourvu d'une première ouverture, permettant le dépôt dans la zone de collection du support solide poreux, du réactif de liaison conjugué à un marqueur particulaire et de l'échantillon, et d'une deuxième ouverture, en amont de la première ouverture, permettant le dépôt dans la zone de collection du support solide poreux, du diluant sous forme liquide.

Préférentiellement, le support de préhension est constitué d'un boîtier.

Par « analyte », on entend toute entité chimique, biochimique ou biologique que l'on souhaite détecter dans un échantillon. Parmi les analytes détectés par les procédés selon la présente invention, on citera notamment les protéines, les peptides, les anticorps, les hormones, les stéroïdes, les antigènes dérivés d'agents infectieux ou de cellules tumorales, les agents infectieux tels que les bactéries, les virus ou les parasites, les acides nucléiques (ADN ou ARN), les molécules thérapeutiques, les drogues ou encore les antibiotiques.

Par « détecter », on entend la détermination de la présence ou de l'absence d'un analyte dans un échantillon mais aussi la mesure et la quantification d'un analyte dans un échantillon. En effet, les performances des procédés selon l'invention autorisent la réalisation de mesures quantitatives ou semi quantitatives.

Dans un mode de réalisation particulier de l'invention, l'analyte est la hCG (hormone choriogonadotropine) ou le PSA (antigène prostatique).

Dans un autre mode de réalisation particulier de l'invention, l'analyte est un acide nucléique. Dans ce cas, l'acide nucléique présent dans l'échantillon est préférentiellement amplifié préalablement selon des techniques bien connues de l'homme du métier (PCR, etc.). De préférence, cette étape d'amplification permet également de marquer l'acide nucléique amplifié en utilisant des amorces biotinylées ou par incorporation de nucléotides marqués à la biotine par exemple. Alternativement, l'acide nucléique peut être, par exemple, marqué à la biotine à son extrémité 3' à l'aide d'une terminale transférase appropriée. Avant le dépôt l'échantillon est dénaturé soit par choc thermique soit en présence d'une solution de NaOH 0,2 N, EDTA 0,05 M ou de toute autre méthode appropriée. Cette étape de dénaturation permet d'obtenir des acides nucléiques simple brin.

Par « échantillon liquide », on entend tout échantillon dans lequel l'analyte recherché est en solution ou en suspension. Cet échantillon liquide peut notamment être tout fluide biologique ou corporel. L'échantillon liquide peut également avoir été obtenu directement ou indirectement à partir d'un fluide biologique ou corporel. L'échantillon peut également être un extrait liquide d'un échantillon solide.

Dans un mode de réalisation préféré de l'invention, l'échantillon liquide est de l'urine, du sang total, du plasma ou du sérum.

Les réactifs mis en oeuvre dans les procédés selon la présente invention sont bien connus de l'homme du métier.

Le réactif de liaison conjugué à un marqueur particulaire et le réactif de capture sont spécifiques de l'analyte recherché dans l'échantillon.

Dans un mode de réalisation particulier de l'invention, le réactif de liaison conjugué à un marqueur particulaire et le réactif de capture immobilisé dans la zone de détection du support solide permettent de détecter l'analyte par un test sandwich.

Dans un autre mode de réalisation particulier de l'invention, le réactif de liaison conjugué à un marqueur particulaire et le réactif de capture spécifique de l'analyte immobilisé dans la zone de détection du support solide permettent de détecter l'analyte par un test de compétition.

Les tests sandwich et les tests par compétition sont bien connus de l'homme du métier. Dans un test sandwich, le réactif de capture spécifique de l'analyte et le réactif de liaison marqué sont prédéterminés pour se lier respectivement et spécifiquement avec l'analyte, par exemple sur deux sites épitopiques, identiques ou différents de l'analyte. Dans un test par compétition, le réactif de liaison marqué est identique ou analogue à l'analyte, pour se lier avec le réactif de capture, en compétition avec l'analyte.

Par « réactif de capture», on entend toute entité chimique biochimique ou biologique susceptible de se lier spécifiquement avec l'analyte.

Dans le cas d'un test par compétition, le réactif de capture se lie également au réactif de liaison. L'analyte et le réactif de capture forment typiquement un couple ligand/anti-ligand, antigène/anticorps, ADN/ARN ou ADN/ADN. Ainsi, si l'analyte est un antigène ou un haptène, le réactif de capture est par exemple un anticorps spécifique de l'analyte. Si l'analyte est un anticorps, le réactif de capture est l'antigène reconnu par l'anticorps ou un anticorps reconnaissant spécifiquement l'analyte. Si l'analyte est un acide nucléique, le réactif de capture est par exemple une sonde ADN complémentaire.

Le réactif de capture immobilisé est préférentiellement un anticorps polyclonal ou monoclonal ayant une forte affinité pour l'analyte et plus préférentiellement il s'agit d'un anticorps monoclonal.

Le réactif de capture spécifique de l'analyte est immobilisé sur le support solide selon des techniques connues de l'homme du métier. Ce réactif de capture est immobilisé de telle façon qu'il ne soit pas mobile à l'état humide. Cette immobilisation peut s'effectuer par exemple par absorption ou par un couplage covalent. Lorsque le réactif de capture est un acide nucléique, il est par exemple fixé sur un support de type nitrocellulose par un traitement UV ou par toute autre technique connue de l'homme du métier.

Par « réactif de liaison », on entend toute entité chimique biochimique ou biologique susceptible de se lier spécifiquement avec l'analyte, ou avec le réactif de capture en compétition avec l'analyte.

Par « lier » ou « liaison », on entend toute liaison forte, par exemple covalente ou toute liaison faible, par exemple du type antigène/anticorps ou avidine/streptavidine.

Le réactif de liaison est par exemple un anticorps, un antigène ou un acide nucléique.

Tout autre réactif de liaison connu de l'homme du métier peut être utilisé tel que un (ou des anticorps) monoclonal aux antibiotine, de l'avidine, de la streptovidine, ou de la polytroptavidine. Ces réactifs peuvent être natifs ou recombinants.

Dans un test par compétition, le réactif de liaison est par exemple l'analyte lui-même ou un analogue approprié de l'analyte. Par analogue approprié de l'analyte, on entend un analogue se liant de manière spécifique au réactif de capture spécifique de l'analyte. Le réactif de liaison marqué est donc l'analyte conjugué à un marqueur particulaire, ou un analogue de l'analyte conjugué à un marqueur particulaire.

Dans un test de type sandwich, le réactif de liaison se lie de façon spécifique à l'analyte. Le réactif de liaison marqué est donc par exemple un anticorps spécifique de l'analyte conjugué à un marqueur particulaire.

Lorsque l'analyte est un acide nucléique marqué à la biotine, le réactif de liaison est typiquement un anticorps anti-biotine conjugué à un marqueur particulaire tel que de l'or colloïdal par exemple.

Le réactif de liaison est conjugué à un marqueur particulaire permettant une mesure ou une observation directe du résultat du test. Le marqueur particulaire peut être observé directement à l'oeil nu lorsqu'il est concentré dans la zone de détection du support solide. La mesure du marqueur particulaire peut s'effectuer directement à l'oeil nu ou à l'aide d'un appareil de mesure. Cette mesure se fait par une observation directe ne nécessitant pas de manipulation supplémentaire. Typiquement, les marqueurs particulaires sont constitués de particules de petite taille insolubles dans l'eau et qui forment donc des suspensions en phase liquide c'est-à-dire une dispersion de particules solides dans un liquide.

Les marqueurs particulaires sont bien connus de l'homme du métier. On connaît notamment les marqueurs particulaires colorés ou fluorescents. A titre d'exemple, on citera l'or colloïdal, les particules de latex colorées, les particules de latex fluorescentes et les particules conjuguées à l'avidine ou à la streptavidine.

Parmi les marqueurs permettant une observation directe à l'oeil nu on citera aussi les marqueurs de type dextran (Hansen T.M., IVD Technology 4, 35-40, 2003). Le réactif de liaison est alors conjugué à une chaîne de dextran (dérivé de polysaccharide) portant des fluorophores.

Le réactif de liaison est conjugué au marqueur particulaire selon des techniques connues.

Dans les procédés selon la présente invention, le réactif de liaison conjugué à un marqueur particulaire est sous forme liquide.

Par « réactif sous forme liquide», on entend tout réactif dans lequel le réactif de liaison est en solution ou en suspension. La préparation du réactif de liaison conjugué au marqueur particulaire sous forme liquide se fait selon des techniques décrites dans la littérature. Habituellement, le réactif de liaison conjugué est en solution ou en suspension dans une solution saline tamponnée. Cette solution peut également comprendre des agents stabilisants et d'autres composés tels que des anti-bactériens ou des anti-fongiques. Parmi les agents stabilisants on citera par exemple la sérum albumine bovine (BSA) et la caséine.

Dans certains procédés selon la présente invention, un diluant est utilisé lorsque l'échantillon liquide est du plasma, du sérum ou du sang total par exemple. Ce diluant migre dans le support solide entraînant l'échantillon et le réactif de liaison marqué. Typiquement ce diluant est composé d'une solution saline tamponnée, il peut également comprendre un détergent ou tout autre composant nécessaire à la réaction.

Dans les procédés de détection d'acides nucléiques, le diluant peut être constitué d'un tampon d'hybridation. De tels tampons d'hybridation sont bien connus de l'homme du métier.

Les supports solides poreux mis en oeuvre dans les tests immunochromatographiques selon l'invention sont bien connus de l'homme du métier (EP 0 284 232). La porosité du support permet la diffusion capillaire de l'échantillon et des réactifs à l'état liquide ou humide.

A titre d'exemple, le support solide poreux peut être constitué de divers supports chromatographiques, de cellulose, de nylon, de nitrocellulose, de polyéthylène ou de fibre de verre.

Le support solide peut être constitué d'une ou de plusieurs parties distinctes. Les différentes parties du support pouvant être constitués de matériaux différents. Lorsque le support solide est constitué de différentes parties ou de différents matériaux, ces éléments sont disposés de telle façon à permettre la continuité de l'écoulement capillaire dans le support solide.

De préférence, le support solide poreux est un support solide poreux en forme de bande ou de bandelette chromatographique.

Le support solide peut ainsi se présenter sous la forme d'une bande chromatographique constituée de plusieurs bandelettes superposés ou chevauchantes.

Typiquement, le support solide poreux comporte une zone de collection de l'échantillon et une zone de détection portant le réactif de capture. Ces zones sont disposées de façon à permettre la continuité de l'écoulement capillaire depuis la zone de collection jusqu'à la zone de détection. La zone de collection et la zone de détection sont deux zones distinctes et séparées du support solide poreux. L'échantillon, le réactif de liaison marqué et le cas échéant le diluant sont déposés au niveau de la zone de collection et migrent à travers le support solide poreux jusqu'à la zone de détection. Le support solide poreux est ainsi par exemple constitué d'une bande chromatographique dont l'une des extrémités constitue la zone de collection, la zone de détection étant située à proximité de l'autre extrémité de la bande.

Ces zones peuvent par exemple être présentes dans un même plan sur une bande constituée d'un matériau unique. Avantageusement, un matériau spécifique correspond à chaque zone du support solide. Un matériau absorbant poreux peut par exemple être utilisé pour la zone de collection de l'échantillon.

La zone de collection de l'échantillon du support solide peut ainsi être constituée d'un organe de captation en matériau absorbant. Cet organe de captation peut être directement mis en contact avec un flux d'urine par exemple. Le support solide peut également comprendre un organe de captation mobile tel que décrit dans WO 00/00288.

La zone de détection du support solide poreux peut également comporter un deuxième réactif de capture immobilisé sur le support poreux en aval du premier réactif de capture. Ce deuxième réactif de capture immobilisé permet de contrôler le bon déroulement du test en vérifiant par exemple la migration du réactif de liaison conjugué au marqueur particulaire dans le support solide. Le deuxième réactif de capture est par exemple un anticorps spécifique du réactif de liaison.

Dans les procédés selon la présente invention, le réactif de liaison marqué, l'échantillon et le cas échéant le diluant sont déposés séparément, successivement, extemporanément et sous forme liquide dans la zone de collection du support solide. Le dépôt extemporané du réactif de liaison marqué sous forme liquide avant l'échantillon et/ou avant le diluant permet de diminuer bruit de fond et effet de zone tout en augmentant la sensibilité grâce au contact immédiat et total entre l'échantillon et le réactif de liaison marqué. La reproductibilité des procédés selon l'invention est également considérablement augmentée par le dosage précis du réactif de liaison marqué ajouté sous forme liquide.

De plus, dans les procédés selon la présente invention le dépôt extemporané du réactif de liaison marqué sous forme liquide avant l'échantillon et/ou avant le diluant permet d'obtenir un effet de lavage qui conduit à une diminution du bruit de fond et de l'effet de zone. Ceci est particulièrement avantageux pour la détection d'analytes dans un échantillon complexe tel que du sang par exemple. De façon avantageuse, l'échantillon ou le diluant est déposé dans la zone de collection en amont du réactif de liaison marqué.

Afin de contrôler la quantité de réactif de liaison marqué déposé sur la zone de collection du support solide poreux, ce dépôt s'effectue de préférence à l'aide d'une pipette, d'un goutte à goutte ou d'un flacon compte-gouttes.

L'échantillon liquide peut également être déposé à l'aide d'une pipette, d'un goutte à goutte ou d'un flacon compte-gouttes. Dans un autre mode de réalisation, le dépôt de l'échantillon est effectué en trempant la zone de collection du support solide dans l'échantillon liquide. Lorsque l'échantillon liquide est de l'urine, la zone de collection du support solide peut aussi être directement mise en contact avec un flux d'urine.

Dans un mode de réalisation préféré de l'invention, le support solide poreux est intégré dans un support de préhension. Ce support de préhension peut envelopper partiellement ou totalement le support solide poreux. Habituellement, le support de préhension est en forme de boîtier.

Ces supports de préhension ou boîtiers sont notamment décrits dans EP 0 291 194, EP 0 560 411, EP 0 560 410 et dans WO 00/00288.

Le support de préhension facilite la manipulation du support solide poreux et protège celui-ci de l'humidité notamment.

Le support de préhension peut être constitué de matériaux divers tel que du carton, du carton plastifié ou plus préférentiellement de matières plastiques. De façon avantageuse, le support de préhension est constitué d'un matériau rigide et imperméable.

Typiquement, le support de préhension est pourvu d'au moins une fenêtre d'observation pour observer la zone de détection du support solide poreux.

Dans un mode de réalisation de l'invention, le support solide poreux peut comprendre une zone de collection saillante par rapport au support de préhension pour le dépôt de l'échantillon liquide et/ou du réactif de liaison marqué et/ou du diluant.

Dans un autre mode de réalisation de l'invention, le support de préhension ou le boîtier comprend au moins une ouverture pour le dépôt de l'échantillon liquide et/ou du réactif de liaison marqué et/ou du diluant dans la zone de collection du support solide poreux.

L'invention sera mieux comprise à l'aide des figures et des exemples ci-dessous :

### Figures

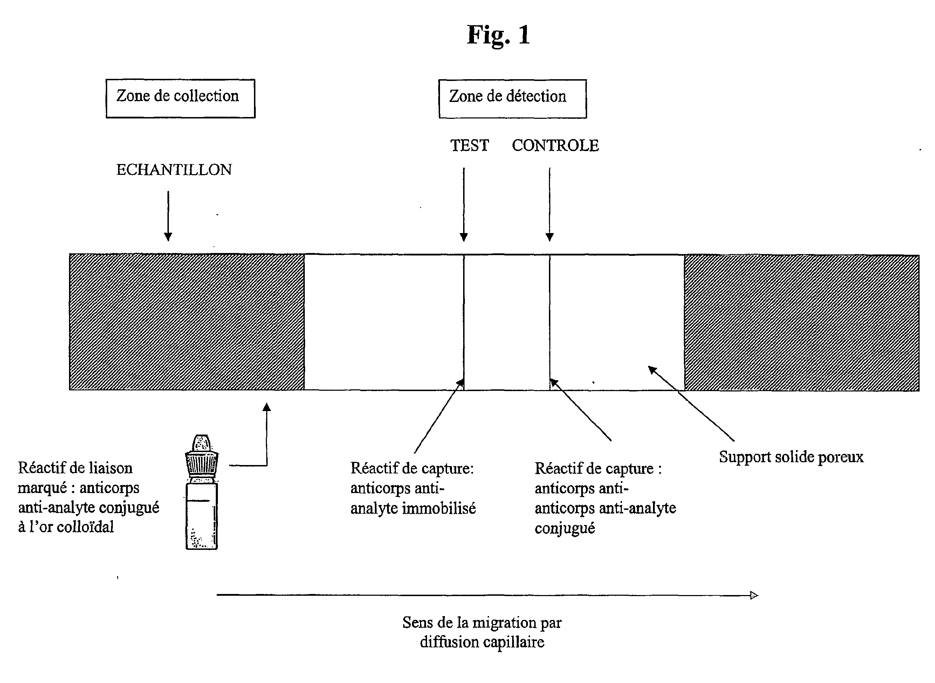

Figure 1 : Principes des procédés immunochromatographiques de l'invention
   Les régions grisées représentent des parties du support solide constituées d'un matériau absorbant.
Figure 2 : Dispositif pour tests immunochromatographiques

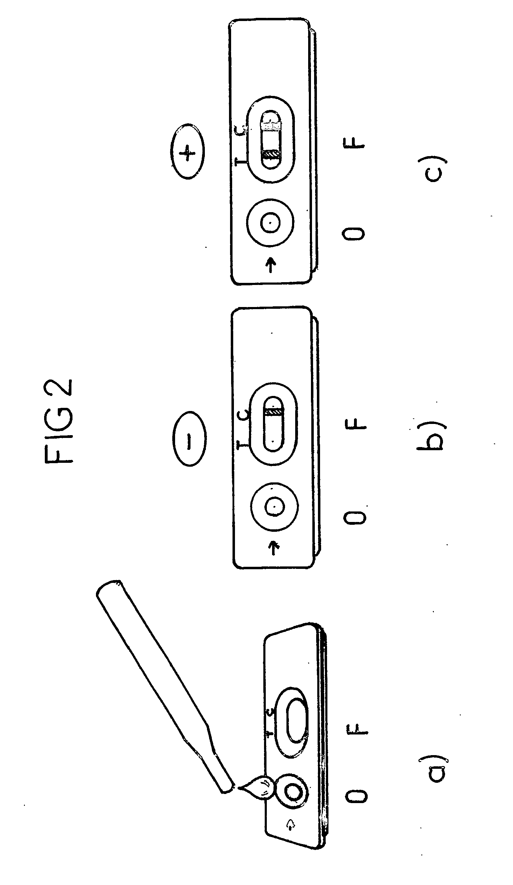

   La figure 2 représente un dispositif comprenant un boîtier comprenant un support solide poreux. Le boîtier est pourvu d'une ouverture (O) pour le dépôt des liquides et d'une fenêtre d'observation (F). La figure 2a représente le dépôt des liquides sur le support solide par l'ouverture dans le boîtier. La figure 2b montre un résultat négatif visible par la fenêtre d'observation (F). La figure 2c montre un résultat positif pour un test sandwich visible par a fenêtre d'observation (F).
   T= ligne de test, C= ligne de contrôle, O = ouverture, F= fenêtre d'observation.

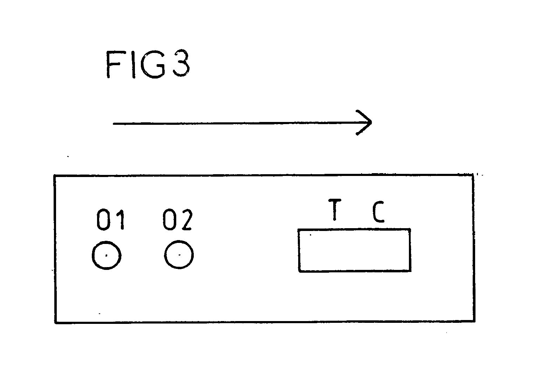

Figure 3 : Dispositif pour tests immunochromatographiques à deux puits
   La figure 3 représente un boîtier comprenant un support solide poreux. Le boîtier est pourvu de deux ouvertures distinctes (01 et 02) pour le dépôt des liquides et d'une fenêtre d'observation. La flèche indique le sens de migration par diffusion capillaire.
   01 = ouverture n° 1, 02 = ouverture n°2, T - ligne de test, C- ligne de contrôle.

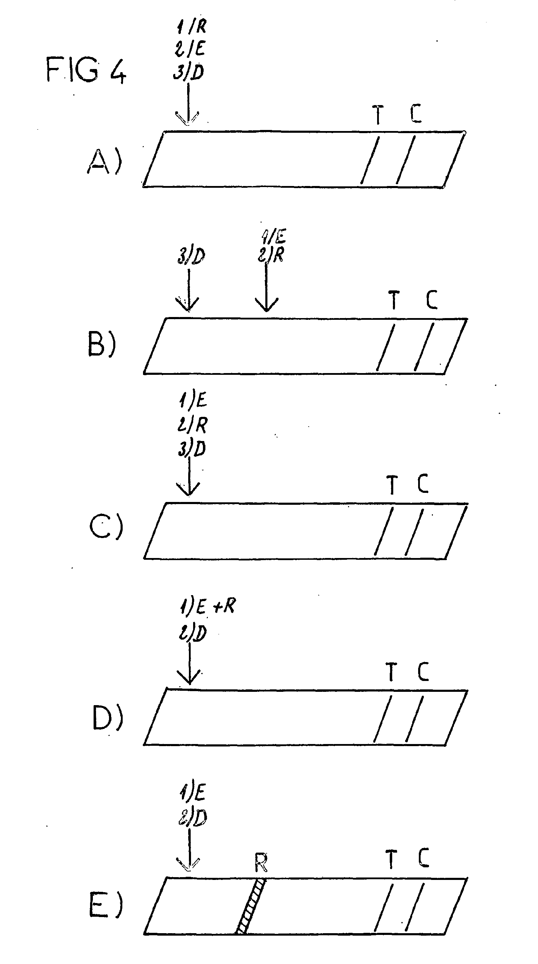

Figure 4 : Exemples comparatifs avec diluant
   R= réactif de liaison marqué, E= échantillon, D = diluant, T = ligne de test, C = ligne de contrôle.

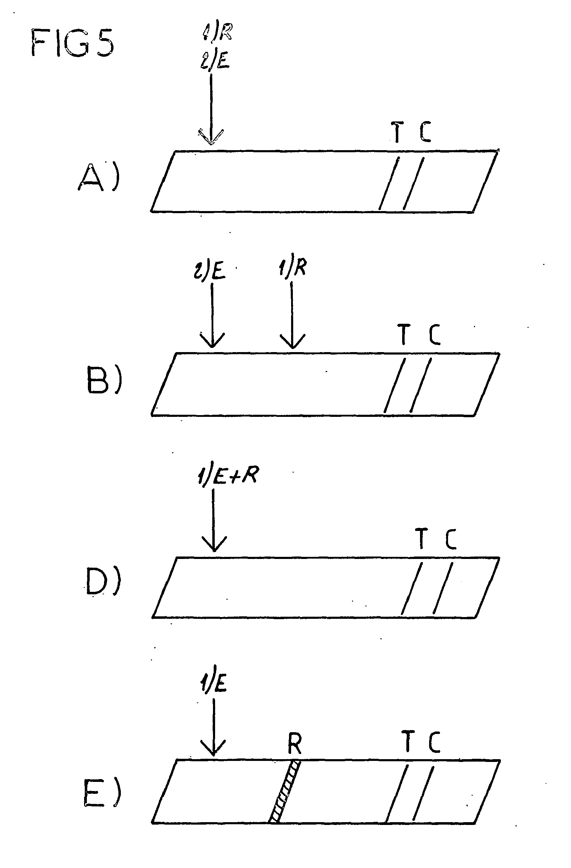

Figure 5 : Exemples comparatifs sans diluant
   R = réactif de liaison marqué, E= échantillon, T = ligne de test, C = ligne de contrôle.

### Exemples

### Exemple 1 : Procédés immunochromatographiques avec diluant

### Dispositif

Les procédés ont été mis en oeuvre avec les dispositifs représentés à la figure 2 et à la figure 3.

### Analyte et échantillon

L'analyte est l'antigène prostatique (PSA) détecté dans du sérum. Le test pourrait être réalisé de la même façon avec du sang total ou du plasma.

### Réactifs et diluant

Le réactif de liaison marqué est un anticorps monoclonal ou polyclonal anti-PSA conjugué avec de l'or colloïdal dans un tampon (PBS 0,1M, pH 8) contenant de la sérum albumine bovine (BSA 1%) comme stabilisant.

Au niveau de la ligne de test de la zone de détection est immobilisé un premier réactif de capture. Ce premier réactif de capture est un anticorps monoclonal ou polyclonal anti-PSA.

Au niveau de la ligne de contrôle de la zone de détection est immobilisé un deuxième réactif de capture. Ce deuxième réactif de capture est un anticorps monoclonal ou polyclonal dirigé contre l'anticorps du réactif de liaison marqué. Le diluant est constitué d'un tampon PBS (0,1 M, pH 8) contenant un détergent (0,05% Tween 20).

### Procédés

Les différents procédés qui ont été comparés sont représentés dans la figure 4. Le procédé A, est conforme à l'invention. Dans tous les cas, la quantité d'échantillon et la quantité de réactif de liaison conjugué au marqueur particulaire par test étaient identiques quelle que soit le procédé considéré pour ne pas fausser les résultats.
Volume échantillon = 25 µl (Version A, B, C, D, E)
Volume diluant = 100 µl (Version A, B, C, D), 150 µl (Version E).
Volume réactif de liaison marqué = 35 µl (Version A, B, C, D), identique mais sous forme déshydratée (Version E).

Les procédés ont été réalisés de la façon suivante :
Version A
   1) Réactif de liaison marqué
   2) Echantillon
   3) Diluant
Version B
   1) Echantillon dans ouverture 2
   2) Réactif de liaison marqué dans ouverture 2
   3) Diluant dans ouverture 1 (en amont de l'ouverture 2).
Version C
   1) Echantillon
   2) Réactif de liaison marqué
   3) Diluant
Version D
   1) Echantillon et réactif de liaison marqué pré-mélangés
   2) Diluant
Version E
   1) Echantillon
   2) Diluant

Dans ce dernier procédé le réactif de liaison marqué est directement porté par le support solide.

### Résultats

Les performances obtenues pour chacun des procédés ont été mesurées et quantifiées à l'aide d'un réflectomètre. L'effet de zone est évalué en utilisant un échantillon très concentré en analyte.

| Procédé | Bruit de fond | Effet de zone | Sensibilité | Reproductibilité |
|---|---|---|---|---|
| A | 4 | 5 | 5 | 4 |
| B | 5 | 3 | 4 | 4 |
| C | 4 | 4 | 3 | 4 |
| D | 4 | 4 | 2 | 4 |
| E | 3 | 2 | 1 | 2 |

Classification de 1 à 5 (1 le moins performant, 5 le plus performant).

### Exemple 2 : Procédés immunochromatographiques sans diluant

### Dispositif

Les procédés ont été mis en oeuvre avec les dispositifs représentés à la figure 2 et à la figure 3.

### Analyte et échantillon

L'analyte est l'hormone choriogonadotropine (hCG) détectée dans de l'urine. Le test pourrait être réalisé de la même façon avec du sérum ou du plasma.

### Réactifs

Le réactif de liaison marqué est un anticorps monoclonal ou polyclonal anti-hCG conjugué avec de l'or colloïdal dans un tampon (PBS 0,1M, pH 8) contenant de la sérum albumine bovine (BSA 1 %) comme stabilisant.

Au niveau de la ligne de test de la zone de détection est immobilisé un premier réactif de capture. Ce premier réactif de capture est un anticorps monoclonal ou polyclonal anti-hCG.

Au niveau de la ligne de contrôle de la zone de détection est immobilisé un deuxième réactif de capture. Ce deuxième réactif de capture est un anticorps monoclonal ou polyclonal dirigé contre l'anticorps du réactif de liaison marqué.

### Procédés

Les différents procédés qui ont été comparés sont représentés dans la figure 5. Les procédés A et B sont conformes à l'invention. Dans tous les cas, la quantité d'échantillon et la quantité de réactif de liaison conjugué au marqueur particulaire par test étaient identiques quel que soit le procédé considéré pour ne pas fausser les résultats.
Volume échantillon = 100 µl (Version A, B, D), 100 µl + 35 µl (Version E)
Volume réactif de liaison marqué = 35 µl (Version A, B, D), identique mais sous forme déshydratée (Version E).

Les procédés ont été réalisés de la façon suivante :
Version A
   1) Réactif de liaison marqué
   2) Echantillon
Version B
   1) Réactif de liaison marqué dans ouverture 2
   2) Echantillon dans ouverture 1
Version D
   1) Echantillon et réactif de liaison marqué pré-mélangés
Version E
   1) Echantillon

Dans ce dernier procédé le réactif de liaison marqué est directement porté par le support solide.

### Résultats

Les performances obtenues pour chacun des procédés ont été mesurées et quantifiées à l'aide d'un réflectomètre. L'effet de zone est évalué en utilisant un échantillon très concentré en analyte.

| Procédé | Bruit de fond | Effet de zone | Sensibilité | Reproductibilité |
|---|---|---|---|---|
| A | 4 | 4 | 4 | 4 |
| B | 5 | 5 | 1 | 4 |
| D | 3 | 1 | 5 | 4 |
| E | 3 | 3 | 3 | 2 |

Classification de 1 à 5 (1 le moins performant, 5 le plus performant).

### Exemple 3 : Procédé de détection de la morphine (test compétition)

### Dispositif

Ce procédé a été mis en oeuvre avec le dispositif représenté à la figure 2.

### Analyte et échantillon

L'analyte est la morphine détectée dans de l'urine.

### Réactifs

Le réactif de liaison marqué est un haptène morphine-BSA conjugué à des particules d'or colloïdal dans un tampon (PBS 0,1M, pH 8) contenant de la sérum albumine bovine (BSA 1%) comme stabilisant.
Au niveau de la ligne de test de la zone de détection est immobilisé un réactif de capture. Ce premier réactif de capture est un anticorps monoclonal anti-morphine.

### Procédé

Dépôt de 35 µl de réactif de liaison conjugué au marqueur particulaire puis dépôt de 150 µl d'urine dans le même puit du boîtier. Lecture des résultats 5 minutes plus tard.

### Résultats

Par rapport à un test mettant en oeuvre un support solide portant le réactif de liaison conjugué sous forme déshydratée, le bruit de fond est diminué et la reproductibilité est améliorée.

### Exemple 4 : Procédé de détection d'un acide nucléique

### Dispositif

Ce procédé est mis en oeuvre avec le dispositif représenté à la figure 2.

Le dispositif comprend une bandelette de nitrocellulose sur laquelle est fixée (par traitement UV ou par toute autre technique) une sonde spécifique d'un acide nucléique d'*E.coli* ou de *Chlamydia.* La bandelette est incluse dans un boîtier en plastique pourvu d'une ouverture pour le dépôt des réactifs et d'une fenêtre d'observation.

### Analyte et échantillon

L'analyte recherché dans l'échantillon est un ADN ou un ARN d' *E.coli* ou de *Chlamydia.* L'acide nucléique est préalablement amplifié selon des méthodes classiques comme par exemple la PCR. Le marquage de l'ADN est réalisé au moyen d'amorces biotinylées ou par incorporation de nucléotides marqués à la biotine. Alternativement, l'extrémité 3' des acides nucléiques est marquée à la biotine à l'aide d'une terminale transférase.

### Réactifs

Le réactif de liaison marqué est un anticorps polyclonal de lapin anti-biotine marqué à l'or colloïdal.

Le tampon d'hybridation est un tampon PBS contenant 0,1 % de Tween 20.

D'autres tampons d'hybridation peuvent être utilisés.

### Procédé

Les acides nucléiques amplifiés et marqués à la biotine (échantillon) sont dénaturés soit par choc thermique, soit en présence de NaOH 1,2N, EDTA 0,05 M.
25 µl d'échantillon dénaturé sont déposés dans le puit échantillon (ouverture).
40 µl de réactif de liaison marqué (conjugué anti-biotine) sont ensuite déposés dans le puit échantillon. Ces deux premières étapes peuvent être inversées.
150 µl à 200 µl de tampon d'hybridation sont ensuite déposés dans le puit échantillon.

### Résultats

Lorsque l'acide nucléique recherché est présent dans l'échantillon il migre par diffusion depuis la zone de collection jusqu'à la zone de détection où il se fixe à la sonde immobilisée sur le support. Une bande rouge apparaît dans la zone test (lecture du test 10 à 20 minutes après les dépôts). La ligne de contrôle apparaît également dans la zone de détection et montre la bonne fonctionnalité des réactifs.

## Revendications

1. Procédé de détection d'un analyte dans un échantillon liquide, comprenant les étapes suivantes:
a) on dispose d'un support solide poreux pourvu d'une zone de collection et d'une zone de détection, un réactif de capture spécifique dudit analyte étant immobilisé dans la zone de détection,
b) on dépose, séparément, successivement et extemporanément, dans la zone de collection du support solide poreux:
i) un réactif de liaison, susceptible de se lier spécifiquement avec l'analyte ou avec le réactif de capture, conjugué à un marqueur particulaire, le réactif étant sous forme liquide,
ii) l'échantillon liquide,
c) on attend un temps suffisant pour la migration par diffusion capillaire du réactif de liaison conjugué à un marqueur particulaire et de l'échantillon liquide, depuis la zone de collection jusqu'à la zone de détection du support solide poreux,
d) on observe le réactif de liaison conjugué à un marqueur particulaire fixé dans la zone de détection.

2. Procédé selon la revendication 1, dans lequel à l'étape b) on dépose l'échantillon liquide en amont du réactif de liaison conjugué à un marqueur particulaire, par rapport au sens de migration depuis la zone de collection jusqu'à la zone de détection du support solide poreux.

3. Procédé selon la revendication 1, dans lequel à l'étape b) on dépose en outre
iii) un diluant sous forme liquide,
et à l'étape c) on attend un temps suffisant pour la migration par diffusion capillaire du réactif de liaison conjugué à un marqueur particulaire, de l'échantillon liquide et du diluant, depuis la zone de collection jusqu'à la zone de détection du support solide poreux.

4. Procédé selon la revendication 3 , dans lequel à l'étape b) on dépose le diluant sous forme liquide en amont du réactif de liaison conjugué à un marqueur particulaire et en amont de l'échantillon liquide, par rapport au sens de migration depuis la zone de collection jusqu'à la zone de détection du support solide poreux.

5. Procédé selon l'une des revendications 1 à 4, dans lequel le réactif de liaison conjugué à un marqueur particulaire et le réactif de capture immobilisé dans la zone de détection permettent de détecter l'analyte par un test sandwich.

6. Procédé selon l'une des revendications 1 à 4, dans lequel le réactif de liaison conjugué à un marqueur particulaire et le réactif de capture immobilisé dans la zone de détection permettent de détecter l'analyte par un test de compétition.

7. Procédé selon l'une des revendications 1 à 6, dans lequel la zone de détection du support solide poreux comporte un deuxième réactif de capture immobilisé en aval du premier réactif de capture.

8. Procédé selon l'une des revendications 1 à 7, dans lequel le support solide poreux est un support solide poreux en forme de bande ou de bandelette chromatographique.

9. Procédé selon l'une des revendications 1 à 8, dans lequel le support solide poreux est intégré dans un support de préhension pourvu d'au moins une fenêtre d'observation permettant d'observer la mesure dans laquelle le réactif conjugué à un marqueur particulaire se fixe dans la zone de détection du support solide poreux.

10. Procédé selon la revendication 9, dans lequel le support de préhension est pourvu d'au moins une ouverture permettant respectivement le dépôt de l'échantillon liquide, du réactif de liaison conjugué à un marqueur et, le cas échéant, du diluant, dans la zone de collection du support solide poreux.

11. Procédé selon la revendication 2, dans lequel le support solide poreux est intégré dans un support de préhension pourvu d'au moins une fenêtre d'observation permettant d'observer la mesure dans laquelle le réactif conjugué à un marqueur particulaire se fixe dans la zone de détection du support solide poreux; le support solide poreux étant également pourvu d'une première ouverture permettant le dépôt dans la zone de collection du support solide poreux, du réactif de liaison conjugué à un marqueur particulaire, et d'une deuxième ouverture, en amont de la première ouverture, permettant le dépôt dans la zone de collection du support solide poreux, de l'échantillon liquide.

12. Procédé selon la revendication 4 dans lequel le support solide poreux est intégré dans un support de préhension pourvu d'au moins une fenêtre d'observation permettant d'observer la mesure dans laquelle le réactif conjugué à un marqueur particulaire se fixe dans la zone de détection du support solide poreux; le support de préhension étant également pourvu d'une première ouverture, permettant le dépôt dans la zone de collection du support solide poreux, du réactif de liaison conjugué à un marqueur particulaire et de l'échantillon, et d'une deuxième ouverture, en amont de la première ouverture, permettant le dépôt dans la zone de collection du support solide poreux, du diluant sous forme liquide.

13. Procédé selon l'une des revendications 9 à 12, dans lequel le support de préhension est constitué d'un boîtier.

## Claims

1. Method for detecting an analyte in a liquid sample, comprising the following steps:
a) a porous solid support provided with a collection zone and a detection zone is provided, a capture reagent specific for said analyte being immobilized in the detection zone;
b) the following are deposited, separately, successively and extemporaneously, in the collection zone of the porous solid support:
i) a binding reagent capable of binding specifically with the analyte or with the capture reagent, conjugated to a particulate label, the reagent being in liquid form,
ii) the liquid sample,
c) an amount of time sufficient for the migration, by capillary diffusion, of the binding reagent conjugated to a particulate label and of the liquid sample from the collection zone to the detection zone of the porous solid support is allowed to elapse,
d) the binding reagent conjugated to a particulate label, attached in the detection zone, is observed.

2. Method according to Claim 1, in which, in step b), the liquid sample is deposited upstream of the binding reagent conjugated to a particulate label, relative to the direction of migration from the collection zone to the detection zone of the porous solid support.

3. Method according to Claim 1, in which, in step b), (iii) a diluent in liquid form is also deposited, and, in step c), an amount of time sufficient for the migration, by capillary diffusion, of the binding reagent conjugated to a particulate label, of the liquid sample and of the diluent, from the collection zone to the detection zone of the porous solid support, is allowed to elapse.

4. Method according to Claim 3, in which, in step b), the diluent in liquid form is deposited upstream of the binding reagent conjugated to a particulate label and upstream of the liquid sample, relative to the direction of migration from the collection zone to the detection zone of the porous solid support.

5. Method according to one of Claims 1 to 4, in which the binding reagent conjugated to a particulate label and the capture reagent immobilized in the detection zone make it possible to detect the analyte by means of a sandwich assay.

6. Method according to one of Claims 1 to 4, in which the binding reagent conjugated to a particulate label and the capture reagent immobilized in the detection zone make it possible to detect the analyte by means of a competition assay.

7. Method according to one of Claims 1 to 6, in which the detection zone of the porous solid support comprises a second capture reagent immobilized downstream of the first capture reagent.

8. Method according to one of Claims 1 to 7, in which the porous solid support is a porous solid support in the form of a chromatographic strip or narrow strip.

9. Method according to one of Claims 1 to 8, in which the porous solid support is integrated into a support to be gripped provided with at least one observation window for observing the extent to which the reagent conjugated to a particulate label attaches in the detection zone of the porous solid support.

10. Method according to Claim 9, in which the support to be gripped is provided with at least one opening for depositing, respectively, the liquid sample, the binding reagent conjugated to a label and, where appropriate, the diluent, in the collection zone of the porous solid support.

11. Method according to Claim 2, in which the porous solid support is integrated into a support to be gripped provided with at least one observation window for observing the extent to which the reagent conjugated to a particulate label attaches in the detection zone of the porous solid support; the porous solid support being also provided with a first opening for depositing the binding reagent conjugated to a particulate label in the collection zone of the porous solid support, and with a second opening, upstream of the first opening, for depositing the liquid sample in the collection zone of the porous solid support.

12. Method according to Claim 4, in which the porous solid support is integrated into a support to be gripped provided with at least one observation window for observing the extent to which the reagent conjugated to a particulate label attaches in the detection zone of the porous solid support; the support to be gripped being also provided with a first opening for depositing the binding reagent conjugated to a particulate label and the sample in the collection zone of the porous solid support, and with a second opening, upstream of the first opening, for depositing the diluent in liquid form in the collection zone of the porous solid support.

13. Method according to one of Claims 9 to 12, in which the support to be gripped consists of a casing.

## Patentansprüche

1. Verfahren zum Nachweis eines Analyten in einer flüssigen Probe, bei dem man:
a) über einen porösen festen Träger verfügt, der mit einer Sammelzone und einer Nachweiszone ausgestattet ist, wobei ein für den Analyten spezifisches Fängerreagenz in der Nachweiszone immobilisiert ist,
b) man in der Sammelzone des porösen festen Trägers getrennt, aufeinander folgend oder unmittelbar vor Gebrauch Folgendes aufbringt:
i) ein Bindungsreagenz, das spezifisch an den Analyten oder an das Fängerreagenz binden kann und mit einem bestimmten Marker konjugiert ist, wobei das Reagenz in flüssiger Form vorliegt,
ii) die flüssige Probe,
c) man eine Zeit lang wartet, die für die Wanderung des mit einem bestimmten Marker konjugierten Bindungsreagenzes und der flüssigen Probe mittels Kapillardiffusion von der Sammelzone bis zur Nachweiszone des porösen festen Trägers ausreicht,
d) man das mit einem bestimmten Marker konjugierte Bindungsreagenz beobachtet, das an die Reaktionszone angelagert wird.

2. Verfahren nach Anspruch 1, bei dem man im Schritt b) die flüssige Probe stromaufwärts, in Bezug auf die Richtung der Wanderung von der Sammelzone bis zur Nachweiszone des porösen festen Trägers, von dem mit einem bestimmten Marker konjugierten Bindungsreagenz aufbringt.

3. Verfahren nach Anspruch 1, wobei man im Schritt b) außerdem
(iii) ein Verdünnungsmittel in flüssiger Form aufbringt,
und man im Schritt c) eine Zeit lang wartet, die für die Wanderung des mit einem bestimmten Marker konjugierten Bindungsreagenzes, der flüssigen Probe und des Verdünnungsmittels mittels Kapillardiffusion von der Sammelzone bis zur Nachweiszone des porösen festen Trägers ausreicht.

4. Verfahren nach Anspruch 3, wobei man im Schritt b) das Verdünnungsmittel in flüssiger Form stromaufwärts, in Bezug auf die Richtung der Wanderung von der Sammelzone bis zur Nachweiszone des porösen festen Trägers, von dem mit einem bestimmten Marker konjugierten Bindungsreagenz und stromaufwärts der flüssigen Probe aufbringt.

5. Verfahren nach einem der Ansprüche 1 bis 4, wobei es das mit einem bestimmten Marker konjugierte Bindungsreagenz und das in der Nachweiszone immobilisierte Fängerreagenz gestatten, den Analyten durch einen Sandwichtest nachzuweisen.

6. Verfahren nach einem der Ansprüche 1 bis 4, wobei es das mit einem bestimmten Marker konjugierte Bindungsreagenz und das in der Nachweiszone immobilisierte Fängerreagenz gestatten, den Analyten durch einen Kompetitionstest nachzuweisen.

7. Verfahren nach einem der Ansprüche 1 bis 6, wobei die Nachweiszone des porösen festen Trägers ein zweites Fängerreagenz trägt, das stromabwärts des ersten Fängerreagenzes immobilisiert ist.

8. Verfahren nach einem der Ansprüche 1 bis 7, wobei der poröse feste Träger ein poröser fester Träger in Form eines Chromatographiestreifens oder -bändchens ist.

9. Verfahren nach einem der Ansprüche 1 bis 8, wobei der poröse feste Träger in einen Handhabungsträger integriert ist, der mit mindestens einem Beobachtungsfenster ausgestattet ist, durch das beobachtet werden kann, in welchem Maße das mit einem bestimmten Marker konjugierte Reagenz in der Nachweiszone des porösen festen Trägers bindet.

10. Verfahren nach Anspruch 9, wobei der Handhabungsträger mit mindestens einer Öffnung versehen ist, die das Aufbringen von jeweils der flüssigen Probe, dem mit einem Marker konjugierten Bindungsreagenz und gegebenenfalls dem Verdünnungsmittels in der Sammelzone des porösen festen Trägers gestattet.

11. Verfahren nach Anspruch 2, wobei der poröse feste Träger in einen Handhabungsträger integriert ist, der mit mindestens einem Beobachtungsfenster ausgestattet ist, durch das beobachtet werden kann, in welchem Maße das mit einem bestimmten Marker konjugierte Reagenz in der Nachweiszone des porösen festen Trägers bindet; wobei der poröse feste Träger ferner mit einer ersten Öffnung versehen ist, die das Aufbringen des mit einem bestimmten Marker konjugierten Bindungsreagenzes in der Sammelzone des porösen festen Trägers gestattet, und mit einer zweiten Öffnung stromaufwärts der ersten Öffnung, die das Aufbringen der flüssigen Probe in der Sammelzone des porösen festen Trägers gestattet.

12. Verfahren nach Anspruch 4, wobei der poröse feste Träger in einen Handhabungsträger integriert ist, der mit mindestens einem Beobachtungsfenster ausgestattet ist, durch das beobachtet werden kann, in welchem Maße das mit einem bestimmten Marker konjugierte Reagenz in der Nachweiszone des porösen festen Trägers bindet; wobei der Handhabungsträger ferner mit einer ersten Öffnung versehen ist, die das Aufbringen des mit einem bestimmten Marker konjugierten Bindungsreagenzes und der Probe in der Sammelzone des porösen festen Trägers gestattet, und mit einer zweiten Öffnung stromaufwärts der ersten Öffnung, die das Aufbringen des Verdünnungsmittels in flüssiger Form in der Sammelzone des porösen festen Trägers gestattet.

13. Verfahren nach einem der Ansprüche 9 bis 12, wobei der Handhabungsträger aus einem Gehäuse besteht.
